(19) **Europäisches Patentamt**
**European Patent Office**
**Office européen des brevets**

(11) **EP 2 163 198 A1**

(12) **EUROPEAN PATENT APPLICATION**

(43) Date of publication:
**17.03.2010 Bulletin 2010/11**

(51) Int Cl.:
***A61B 5/083*** (2006.01)

(21) Application number: **08164033.6**

(22) Date of filing: **10.09.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Nederlandse Organisatie voor toegepast-natuurwetenschappelijk onderzoek TNO**
**2628 VK Delft (NL)**

(72) Inventors:
• **Elsten, Tom Johannes Marinus Maria**
**5654 DB Eindhoven (NL)**
• **Hoppenbrouwers, Marcus Benedictus**
**5611 JX Eindhoven (NL)**
• **Wijnen, Merijn**
**5613 CN Eindhoven (NL)**

(74) Representative: **Hatzmann, Martin**
**Vereenigde**
**Johan de Wittlaan 7**
**2517 JR Den Haag (NL)**

(54) **Method and system for quantitative determination of a component in the air exhaled by a user**

(57) Method for quantitative determination of a component, e.g. $CO_2$ and/or $O_2$, in the air exhaled by a user, comprising collecting an air sample containing the exhaled air and ambient air and determining the relative humidity RHs of the air sample. Next, assessing a factor p according to the formula (I) p = (RHs - RHa) / (RHe - RHa), where RHs is the determined humidity of the air sample, RHa is the presumed humidity of the ambient air and RHe of the exhaled air. Then determining the percentage Cs of said component in the air sample, followed by assessing the percentage Ce of said component to be assigned to the exhaled air by applying the formula (II) Ce = ((Cs - Ca) / p) + Ca, where Cs is the determined percentage of said component in the air sample and Ca is the presumed percentage of the component in the ambient air. A system for performing the method comprises collection means (1) for collecting an air sample containing exhaled air and ambient air, means (2) for determining the humidity RHs of the collected air sample, means (3) for assessing said factor p according to formula I, means (4, 6) for determining the percentage Cs of said component in the air sample, and means (3) for assessing the percentage Ce of said component to be assigned to the exhaled air from formula II. The collection means (1) may comprise an exhalation inlet (7), to be placed about in front of the user's mouth, and one or more exhalation outlets (8), pointing into the same direction as the exhalation inlet.

Fig. 1

**Description**

The present invention refers to quantitative determination of a component like oxygen ($O_2$), carbon dioxide ($CO_2$) etc. in the air exhaled by a user, e.g. an athlete, often indicated as ergospirometry.

Ergospirometry tests relate to concentrations of components such as oxygen ($O_2$) and carbon dioxide ($CO_2$), from in- and expired breath, to the physical capacities of e.g. an athlete. Many tests are done under laboratory conditions. Measuring an athlete under sport specific conditions would of course give more representative results.

Mobile measurement systems exist; their limitation is the fact that they use a mask or mouth piece to capture and analyze the in- and expired air. Masks (i.e. largely the user's mouth/nose) are considered to be awkward and restrain an athlete in his natural breathing pattern.

One aim of this invention is to provide maskless measuring by means of a novel method and system for sampling and processing the expired air of a user (e.g. a sportsman/woman) which enables the use of a dedicated housing for capturing the expired air. In this way various breathing properties of an athlete can be measured while performing real sports activities.

Another aim is to provide a maskless (e.g.) $O_2$/$CO_2$ measurement system that can accurately measure breath gas concentrations while moving at a speeds of up to 15 m/s; the system aims to be able to account for head winds of up to wind force 4 (6 m/s).

Yet another aim is to provide a compact and convenient system in order to minimize any restrictions to the athlete.

The invention is largely based on the understanding that when a mixture of exhalation air and ambient air ("false air") could be used for sensing the relevant air component ($O_2$ or $CO_2$) and the mixture relation of the collected exhalation air and ambient air (or, in other words, the percentage of exhalation air in the air mixture) could be determined, this would open the possibility to use a maskless exhalation air collector, i.e. an air collector which does not need to be protected against inflow of ambient air. According to the invention said mixture relation of the exhalation and ambient air or percentage of exhalation air in the air mixture is derived from the measured relative humidity (RH) of the air mixture, where the RH of the exhalation air and the RH of the ambient air are estimated or measured (presumed or predetermined) beforehand. An interesting point regarding to this is that the RH of the exhalation air has a rather constant value, lying at about 100% (exact values are known from the literature) while the RH of the ambient air can easily be measured.

The present invention thus refers to a method for quantitative determination of a component (e.g. ($O_2$ or $CO_2$) in the air exhaled by a user, comprising next steps:

- collecting an air sample containing air exhaled by the user and ambient air (surrounding the user) in an unknown ratio,
- determining the relative humidity RHs of the collected air sample;
- assessing a factor p representing the relative contribution of the exhalation air in the collected air sample, based on relative humidity values according to formula I:

$$p = (RHs - RHa) / (RHe - RHa)$$

where
RHs is the determined relative humidity of the air sample,
RHa is the measured or predetermined relative humidity of the ambient air and
RHe is the presumed or predetermined relative humidity of the exhaled air;
- determining (e.g. measured by a relevant sensor) the percentage (or amount) Cs of said component in the air sample;
- assessing the percentage Ce of said component to be assigned to the air exhaled by the user from formula II:

$$Ce = ((Cs - Ca) / p) + Ca$$

where
Cs is the determined percentage of said component in the air sample,
Ca is the presumed or predetermined percentage of the component in the ambient air.

So, in short, the percentage p of exhalation air in the air mixture is determined by
p = (RHs - RHa) / (RHe - RHa) (I), where RHs is measured (e.g. by a sensor in the air collector), RHe is presumed (e.g. equal to ~100%) or predetermined while RHa is measured. Then the amount (or percentage) of the relevant component (e.g. $O_2$ or $CO_2$) assigned to the exhalation air can be calculated by Ce = ((Cs - Ca) / p) + Ca (II) where Cs is measured (e.g. by an $O_2$ or $CO_2$ sensor respectively). Ca is estimated or measured (presumed or predetermined) beforehand as - like the values for RHe and RHa - the value for Ca has a rather constant value (ambient air contains about 0% $CO_2$ and about 20% $O_2$).

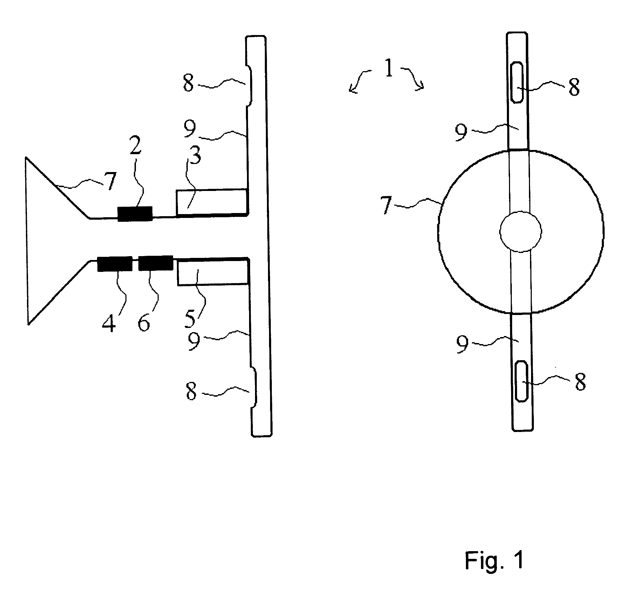

Figure 1 shows an exemplary embodiment in two views of a system which is arranged to perform the method according to invention.

The exemplary embodiment of the system for performing the method discussed above comprises collection means for collecting an air sample containing air exhaled by a user and ambient air in an unknown ratio.

In figure 1 those air sample collection means are formed by an air collector (or air sampler) 1 which can be put before the user's mouth/nose area (not shown), at a certain distance, thus preventing that the collector hinders the user's breathing. At least a substantial part of the user's exhalation air is collected by the collector 1. Due to air turbulences etc. the air collector does not only collect the user's exhalation air, but also some ambient air; so the air sample contains an unknown percentage of air exhaled by the user and an unknown percentage of ambient air in an unknown ratio.

In order to be able to determine the $CO_2$ percentage of (only) the exhalation air in the air sample, the collector 1 comprises means for determining the relative humidity RHs of the collected air sample, formed by an RH sensor 2. Processing means, in the form of a processor 3, are provided for assessing a factor p, representing the relative contribution of the exhalation air in the collected air sample, based on relative humidity values according to the formula (I)

$$p = (RHs - RHa) / (RHe - RHa),$$

where RHs is the determined relative humidity of the air sample, i.e. measured by the RH sensor 2. RHa is the predetermined or measured relative humidity of the ambient air (~20%) and RHe is the presumed relative humidity of the exhaled air (~100%). Instead of using presumed RH values for the ambient this RH value could be determined beforehand, either by means of the same air collector 1 or another one, after which the RHa and RHe values could be input into the processor 3.

The collector 1, moreover, comprises means for determining (measuring) the percentage Cs of $CO_2$ in the air sample, which is formed by a $CO_2$ sensor 4. The processor 3 is also used as means for assessing the $CO_2$ percentage Ce which has to be assigned to the air exhaled by the user, based on the RH of the air sample, measured by RH sensor 2, and the predetermined/presumed RHa and RHe values, according to the formula (II)

$$Ce = ((Cs - Ca) / p) + Ca,$$

where Ce is the calculated $CO_2$ percentage in the exhaled air, Cs is the determined (measured) $CO_2$ percentage in the air sample and Ca is the presumed or predetermined (e.g. measured beforehand using the same $CO_2$ sensor 4 or another $CO_2$ sensor) $CO_2$ percentage in the ambient air (which is about 0% under most conditions).

In this way the requested $CO_2$ percentage in the exhaled air is determined based on the measured RH and the $CO_2$ percentage of the air sample, which is a mixture of exhalation air and ambient air. The thus determined $CO_2$ percentage in the exhaled air could be transmitted (since the athlete will be running, cycling etc.) by means of a radio transmitter 5 to a logging and/or processing system in e.g. a following car.

The $O_2$ percentage or percentage of other components in the exhalation air can be determined in the same way. In that case the $CO_2$ sensor 4 has to be replaced by an $O_2$ sensor or an $O_2$ sensor 6 is added to the collector 1, as shown in figure 1. Processing of the $O_2$ related values can also be performed by the processor 3. It is presumed that the $O_2$ percentage in ambient air (Cs for $O_2$) is 20%). Of course by adding other gas sensors, other breath components can be measured.

Some examples will be given below:

Hereinafter, the relative humidity of the ambient air, RHa, is presumed to be 20%, while the relative humidity of the exhaled air, RHe, is presumed to be 100%.

    A. The measured relative humidity of the air sample is 60%;

        Formula I: p = (60 - 20) / (100 - 20) = 40/80 = ½ (i.e. 50% exhalation air is present in the air sample);
        Measured by sensor 4: 15% $CO_2$ in the air sample
        Formula II: Ce = (15 - 0)/½ + 0 = 30% $CO_2$ in the exhalation air.
        Measured by sensor 6: 18% $O_2$ in the air sample
        Formula II: Ce = (18 - 20)/½ + 20 = -4 + 20 = 16% $O_2$ in the exhalation air.

    B. The measured relative humidity of the air sample is 80%;

        Formula I: p = (80 -20) / (100 - 20) = 60/80 = ¾ (i.e. 75% exhalation air is present in the air sample);
        Measured by sensor 4: 15% $CO_2$ in the air sample (same as in A).
        Formula II: Ce = (15 - 0)/¾ + 0 = 20% $CO_2$ in the exhalation air.
        Measured by sensor 6: 18% $O_2$ in the air sample (same as in A).
        Formula II: Ce = (18 - 20)/¾ + 20 = -1½ + 20 = 18½ % $O_2$ in the exhalation air.

    C. The measured relative humidity of the air sample is 100%;

        Formula I: p = (100 - 20) / (100 - 20) = 1 (i.e. 100% exhalation air is present in the air sample);
        Measured by sensor 4: 15% $CO_2$ in the air sample (same as in A).
        Formula II: Ce = (15 - 0) / 1 + 0 = 15% $CO_2$ in the exhalation air.

Measured by sensor 6: 18% $O_2$ in the air sample (same as in A).
Formula II: $Ce = (18 - 20)/ 1 + 20 = -2 + 20 = 18$ % $O_2$ in the exhalation air.

Note: In example C there is apparently no inflow of ambient air; as a result of this the measured $CO_2$ and $O_2$ percentages by the sensors rightly are fully assigned to the exhalation air.

In figure 1 the exemplary collector 1 comprises an exhalation inlet 7, in operation to be placed about in front of the user's mouth/nose, preferably in a position wherein the user will be least hindered by the collector. To prevent breathing restriction one or more exhalation outlets 8 are provided, connected to the exhalation inlet 7 via connection members 9. The exhalation outlets 8 preferably point into the same direction, (see figure) contrary to the user's movement direction, causing that the flow of exhalation air through the collector is promoted by the draught which occurs at the outlets 8 due to the slip stream of the ambient air during movement of the user/athlete. If desired, a small exhaust fan or the like could be provided to support or boost the flow of exhalation air.

**Claims**

1. Method for quantitative determination of a component in the air exhaled by a user, comprising the following steps:

    - collecting an air sample containing air exhaled by the user and ambient air in an unknown ratio,
    - determining the relative humidity RHs of the collected air sample;
    - assessing a factor p representing the relative contribution of the exhalation air in the collected air sample, based on relative humidity values according to formula I:

$$p = (RHs - RHa) / (RHe - RHa)$$

    where
    RHs is the determined relative humidity of the air sample,
    RHa is the measured or predetermined relative humidity of the ambient air and
    RHe is the presumed or predetermined relative humidity of the exhaled air;
    - determining the percentage Cs of said component in the air sample;
    - assessing the percentage Ce of said component to be assigned to the air exhaled by the user from formula II:

$$Ce = ((Cs - Ca) / p) + Ca$$

    where
    Cs is the determined percentage of said component in the air sample,
    Ca is the presumed or predetermined percentage of the component in the ambient air.

2. Method according to claim 1, said component being $CO_2$.

3. Method according to claim 1, said component being $O_2$.

4. System for performing the method according to claim 1, comprising collection means (1) for collecting an air sample containing air exhaled by the user and ambient air in an unknown ratio, means (2) for determining the relative humidity RHs of the collected air sample, means (3) for assessing said factor p according to said formula I, means (4, 6) for determining the percentage Cs of said component in the air sample, and means (3) for assessing the percentage Ce of said component to be assigned to the air exhaled by the user from said formula II.

5. System according to claim 2, said collection means (1) comprising an exhalation inlet (7), in operation to be placed about in front of the user's mouth, and one or more exhalation outlets (8), connected to the exhalation inlet and pointing into the same direction as the exhalation inlet.

Fig. 1

**EUROPEAN SEARCH REPORT**

Application Number

EP 08 16 4033

Europäisches
Patentamt
European
Patent Office
Office européen
des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 0 196 396 A (COSMED SRL [IT]) 8 October 1986 (1986-10-08) * claim 1; figure 1 * ----- | 1 | INV. A61B5/083 |
| A | WO 98/53732 A (COSMED SRL [IT]; BRUGNOLI PAOLO [IT]) 3 December 1998 (1998-12-03) * claim 1; figure 1 * ----- | 1 | |

TECHNICAL FIELDS
SEARCHED        (IPC)

A61B
A63B

The present search report has been drawn up for all claims

| Place of search | Date of completion of the search | Examiner |
|---|---|---|
| Munich | 29 January 2009 | Schindler, Martin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.......................................................................................
& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 08 16 4033

This annex lists the patent family members relating to the patent documents cited in the above-mentioned European search report.
The members are as contained in the European Patent Office EDP file on
The European Patent Office is in no way liable for these particulars which are merely given for the purpose of information.

29-01-2009

| Patent document cited in search report | | Publication date | Patent family member(s) | | Publication date |
|---|---|---|---|---|---|
| EP 0196396 | A | 08-10-1986 | AT | 59956 T | 15-02-1991 |
| | | | DE | 3581397 D1 | 21-02-1991 |
| | | | US | 4658832 A | 21-04-1987 |
| WO 9853732 | A | 03-12-1998 | EP | 0944348 A1 | 29-09-1999 |
| | | | IT | RM970314 A1 | 27-11-1998 |
| | | | JP | 2001500042 T | 09-01-2001 |
| | | | US | 6206837 B1 | 27-03-2001 |

EPO FORM P0459

For more details about this annex : see Official Journal of the European Patent Office, No. 12/82